# EUROPEAN PATENT SPECIFICATION

(11) **EP 2 741 683 B1**
(45) Date of publication and mention of the grant of the patent: **24.07.2019**
(21) Application number: 12750940.4
(22) Date of filing: 10.08.2012
(51) Int. Cl.: A61B 17/064

(54) **SURGICAL STAPLE WITH LOCALIZED ADJUNCT COATING**
CHIRURGISCHE KLAMMER MIT LOKALISIERTER ZUSATZBESCHICHTUNG
AGRAFE CHIRURGICALE AVEC REVÊTEMENT AUXILIAIRE LOCALISÉ

(30) Priority: 10.08.2011 US 201113206725
(43) Date of publication of application: 18.06.2014
(73) Proprietor: Ethicon Endo-Surgery, Inc., Cincinnati, OH 45242 (US)
(72) Inventor: BEAR, Brian W., Cincinnati, OH 45241 (US); LE, Thu Anh, Bridgewater, NJ 08807 (US); OVERMYER, Mark D., Cincinnati, OH 45236 (US); SETSER, Michael, Burlington, KY 41005 (US); WOODARD, James A., Jr., Mason, OH 45040 (US)
(74) Representative: Carpmaels & Ransford LLP
(86) International application number: PCT/US2012/050226
(87) International publication number: WO 2013/023111

(56) References cited:
- US-A- 6 015 417
- US-A1- 2004 254 608
- US-A1- 2011 064 785
- US-B1- 6 805 898

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in; US 2011/0064785 and U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,964,363, entitled "Surgical Stapling Instrument having Articulation Joint Support Plates for Supporting a Firing Bar," issued November 15, 2005; U.S. Pat. No. 6,978,921, entitled "Surgical Stapling Instrument Incorporating an E-Beam Firing Mechanism," issued December 27, 2005; U.S. Pat. No. 6,988,649, entitled "Surgical Stapling Instrument Having a Spent Cartridge Lockout," issued January 24, 2006; U.S. Pat. No. 7,000,818, entitled "Surgical Stapling Instrument Having Separate Distinct Closing and Firing Systems," issued February 21, 2006; U.S. Pat. No. 7,111,769, entitled "Surgical Instrument Incorporating an Articulation Mechanism having Rotation about the Longitudinal Axis," issued September 26, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument having Multistroke Firing with Opening Lockout," issued October 14, 2008; U.S. Pat. No. 7,721,930, entitled "Disposable Cartridge with Adhesive for Use with a Stapling Device," issued May 25, 2010; and U.S. Pat. No. 7,455,208, entitled "Surgical Instrument with Articulating Shaft with Rigid Firing Bar Supports," issued November 25, 2008. While the surgical staplers referred to above are described as being used in endoscopic procedures, it should be understood that such surgical staplers may also be used in open procedures and/or other non-endoscopic procedures.

The invention is defined by claim 1 and claim 11 and by the appended claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1A depicts a perspective view of an articulating surgical instrument with an end effector in a nonarticulated position;
FIG. 1B depicts a perspective view of the surgical instrument of FIG. 1A with an end effector in an articulated position;
FIG. 2 depicts a perspective view of an opened end effector of the surgical instrument of FIGS. 1A-1B;
FIG. 3A depicts a side cross-sectional view of the end effector of FIG. 2, taken along line 3-3 of FIG. 2, with the firing bar in a proximal position;
FIG. 3B depicts a side cross-sectional view of the end effector of FIG. 2, taken along line 3-3 of FIG. 2, but showing the firing bar in a distal position;
FIG. 4 depicts an end cross-sectional view of the end effector of FIG. 2, taken along line 4-4 of FIG. 2;
FIG. 5 depicts an exploded perspective view of the end effector of FIG. 2;
FIG. 6 depicts a perspective view of the end effector of FIG. 2, positioned at tissue and having been actuated once in the tissue;
FIG. 7 depicts a perspective view of a surgical staple according to the invention with a localized adjunct coating on an interior surface;
FIG. 8 depicts a first example of a cross-sectional view of the coated staple of FIG. 7 along line 8-8;
FIG. 9 depicts a second example of a cross-sectional view of a coated staple;
FIG. 10 depicts the coated staple of FIG. 7 driven into and retaining compressed tissue; and
FIG. 11 depicts a flow chart of an exemplary method for coating an interior of a staple with localized adjunct coating.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention as defined in the appended claims. Accordingly, the drawings and descriptions should be regarded as illustrative in nature and not restrictive.

### I. Exemplary Surgical Stapler

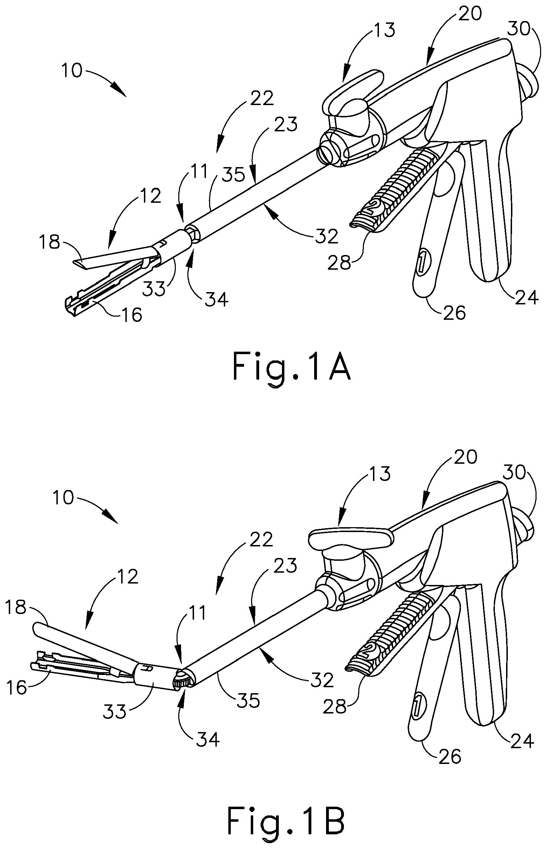

FIGS. 1-6 depict an exemplary surgical stapling and severing instrument (10) that is sized for insertion, in a nonarticulated state as depicted in FIG. 1A, through a trocar cannula passageway to a surgical site in a patient for performing a surgical procedure. Surgical and stapling and severing instrument (10) includes handle portion (20) connected to implement portion (22), the latter further comprising shaft (23) distally terminating in an articulating mechanism (11) and a distally attached end effector (12). Once articulation mechanism (11) and distally end effector (12) are inserted through the cannula passageway of a trocar, articulation mechanism (11) may be remotely articulated, as depicted in FIG. 1B, by articulation control (13). Thereby, end effector (12) may reach behind an organ or approach tissue from a desired angle or for other reasons. It should be understood that terms such as "proximal" and "distal" are used herein with reference to a clinician gripping handle portion (20) of instrument (10). Thus, end effector (12) is distal with respect to the more proximal handle portion (20). It will be further appreciated that for convenience and clarity, spatial terms such as "vertical" and "horizontal" are used herein with respect to the drawings. However, surgical instruments are used in many orientations and positions, and these terms are not intended to be limiting and absolute.

End effector (12) of the present example includes a lower jaw (16) and a pivotable anvil (18). Handle portion (20) includes pistol grip (24) toward which closure trigger (26) is pivotally drawn by the clinician to cause clamping, or closing, of the anvil (18) toward lower jaw (16) of end effector (12). Such closing of anvil (18) is provided through an outmost closure sleeve (32), which longitudinally translates relative to handle portion (20) in response to pivoting of closure trigger (26) relative to pistol grip (24). A distal closure ring (33) of closure sleeve (32) is indirectly supported by frame (34) of implement portion (22). At articulation mechanism (11), a proximal closure tube (35) of closure sleeve (32) communicates with the distal portion (closure ring) (33). Frame (34) is flexibly attached to lower jaw (16) via articulation mechanism (11), enabling articulation in a single plane. Frame (34) also longitudinally slidingly supports a firing drive member (not shown) that extends through shaft (23) and communicates a firing motion from firing trigger (28) to firing bar (14). Firing trigger (28) is farther outboard of closure trigger (26) and is pivotally drawn by the clinician to cause the stapling and severing of clamped tissue in end effector (12), as will be described in greater detail below. Thereafter, release button (30) is depressed to release the tissue from end effector (12).

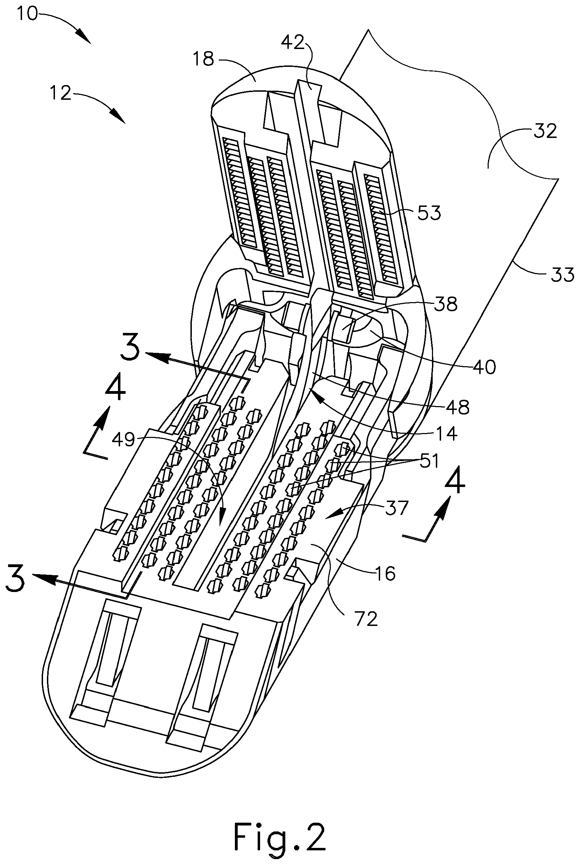

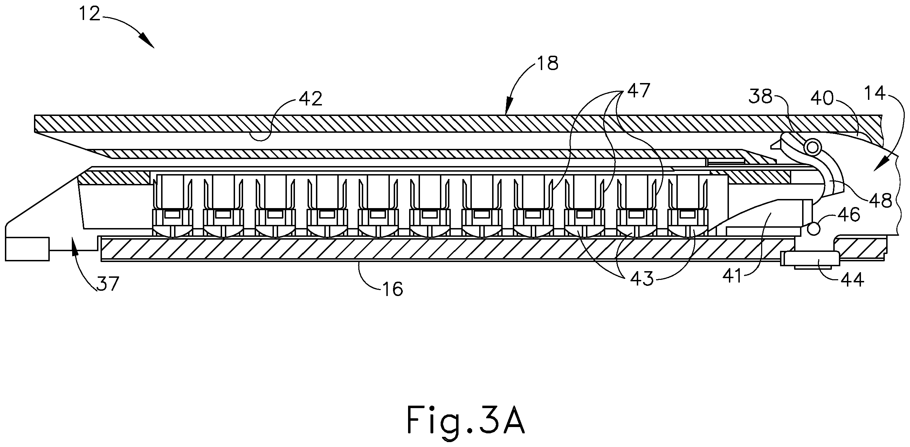

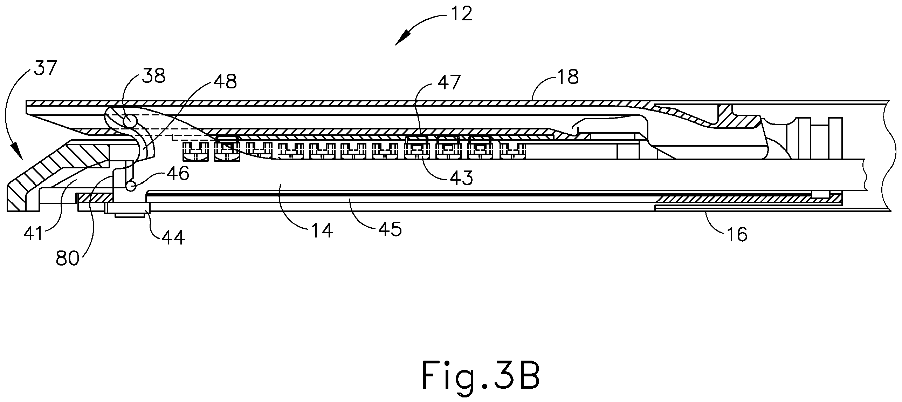

FIGS. 2-5 depict end effector (12) employing an E-beam firing bar (14) to perform a number of functions. As best seen in FIGS. 3A-3B, firing bar (14) includes a transversely oriented upper pin (38), a firing bar cap (44), a transversely oriented middle pin (46), and a distally presented cutting edge (48). Upper pin (38) is positioned and translatable within an anvil pocket (40) of anvil (18). Firing bar cap (44) slidably engages a lower surface of lower jaw (16) by having firing bar (14) extend through channel slot (45) (shown in FIG. 3B) that is formed through lower jaw (16). Middle pin (46) slidingly engages a top surface of lower jaw (16), cooperating with firing bar cap (44). Thereby, firing bar (14) affirmatively spaces end effector (12) during firing, overcoming pinching that may occur between anvil (18) and lower jaw (16) with a minimal amount of clamped tissue and overcoming staple malformation with an excessive amount of clamped tissue.

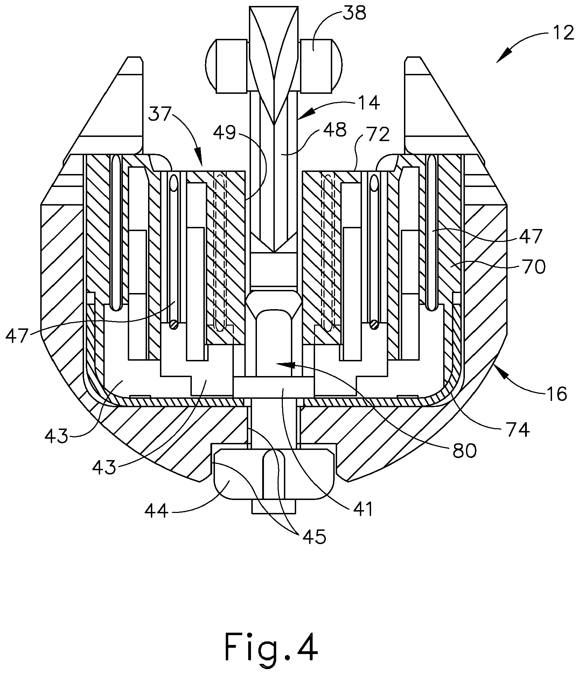

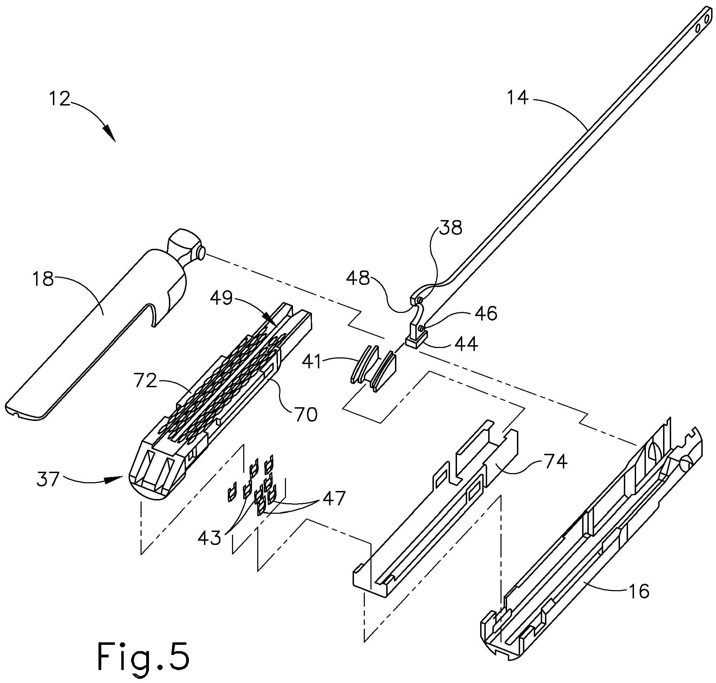

FIG. 2 shows firing bar (14) proximally positioned and anvil (18) pivoted to an open position, allowing an unspent staple cartridge (37) to be removably installed into a channel of lower jaw (16). As best seen in FIGS. 4-5, staple cartridge (37) of this example includes a cartridge body (70), which presents an upper deck (72) and is coupled with a lower cartridge tray (74). As best seen in FIG. 2, a vertical slot (49) is formed through part of staple cartridge (37). As also best seen in FIG. 2, three rows of staple apertures (51) are formed through upper deck (70) on one side of vertical slot (49), with another set of three rows of staple apertures (51) being formed through upper deck (70) on the other side of vertical slot (49). Referring back to FIGS. 3-5, a wedge sled (41) and a plurality of staple drivers (43) are captured between cartridge body (70) and tray (74), with wedge sled (41) being located proximal to staple drivers (43). Wedge sled (41) is movable longitudinally within staple cartridge (37); while staple drivers (43) are movable vertically within staple cartridge (37). Staples (47) are also positioned within cartridge body (70), above corresponding staple drivers (43). In particular, each staple (47) is driven vertically within cartridge body (70) by a staple driver (43) to drive staple (47) out through an associated staple aperture (51). As best seen in FIGS. 3A-3B and 5, wedge sled (41) presents inclined cam surfaces that urge staple drivers (43) upwardly as wedge sled (41) is driven distally through staple cartridge (37).

With end effector (12) closed as depicted in FIG. 3A, firing bar (14) is advanced in engagement with anvil (18) by having upper pin (38) enter a longitudinal anvil slot (42). A pusher block (80) is located at the distal end of firing bar (14), and is configured to engage wedge sled (41) such that wedge sled (41) is pushed distally by pusher block (80) as firing bar (14) is advanced distally through staple cartridge (37). During such firing, cutting edge (48) of firing bar (14) enters vertical slot (49) of staple cartridge (37), severing tissue clamped between staple cartridge (37) and anvil (18). As shown in FIGS. 3A-3B, middle pin (46) and pusher block (80) together actuate staple cartridge (37) by entering into a firing slot within staple cartridge (37), driving wedge sled (41) into upward camming contact with staple drivers (43) that in turn drive staples (47) out through staple apertures (51) and into forming contact with staple forming pockets (53) on the inner surface of anvil (18). FIG. 3B depicts firing bar (14) fully distally translated after completing severing and stapling tissue.

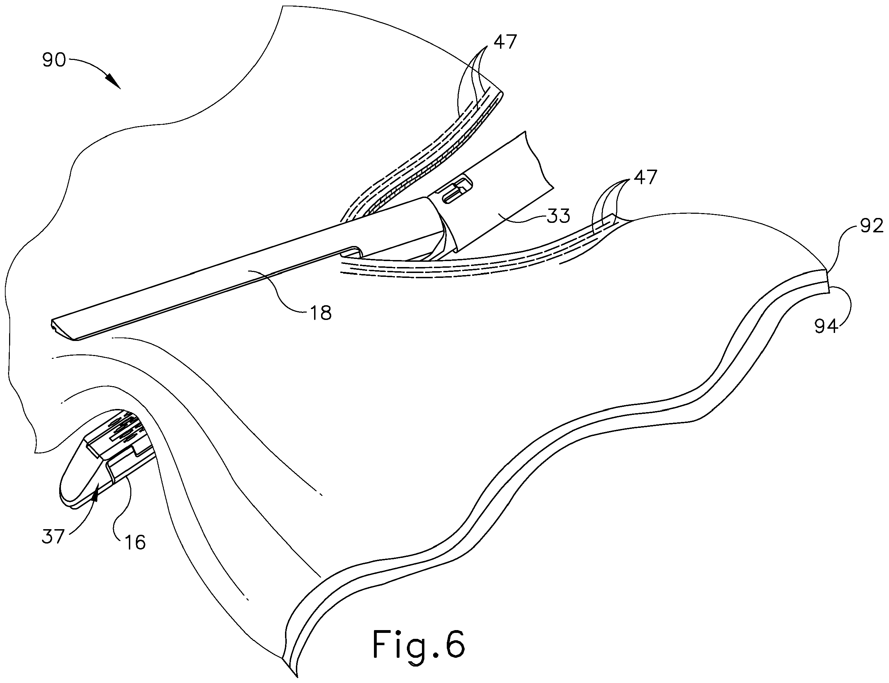

FIG. 6 shows end effector (12) having been actuated through a single stroke through tissue (90). As shown, cutting edge (48) has cut through tissue (90), while staple drivers (43) have driven three alternating rows of staples (47) through the tissue (90) on each side of the cut line produced by cutting edge (48). Staples (47) are all oriented substantially parallel to the cut line in this example, though it should be understood that staples (47) may be positioned at any suitable orientations. In the present example, end effector (12) is withdrawn from the trocar after the first stroke is complete, spent staple cartridge (37) is replaced with a new staple cartridge, and end effector (12) is then again inserted through the trocar to reach the stapling site for further cutting and stapling. This process may be repeated until the desired amount of cuts and staples (47) have been provided. Anvil (18) may need to be closed to facilitate insertion and withdrawal through the trocar; and anvil (18) may need to be opened to facilitate replacement of staple cartridge (37).

It should be understood that cutting edge (48) may sever tissue substantially contemporaneously with staples (47) being driven through tissue during each actuation stroke. In the present example, cutting edge (48) just slightly lags behind driving of staples (47), such that a staple (47) is driven through the tissue just before cutting edge (48) passes through the same region of tissue, though it should be understood that this order may be reversed or that cutting edge (48) may be directly synchronized with adjacent staples. While FIG. 6 shows end effector (12) being actuated in two layers (92, 94) of tissue (90), it should be understood that end effector (12) may be actuated through a single layer of tissue (90) or more than two layers (92, 94) of tissue. It should also be understood that the formation and positioning of staples (47) adjacent to the cut line produced by cutting edge (48) may substantially seal the tissue at the cut line, thereby reducing or preventing bleeding and/or leaking of other bodily fluids at the cut line. Various suitable settings and procedures in which instrument (10) may be used will be apparent to those of ordinary skill in the art in view of the teachings herein.

It should be understood that instrument (10) may be configured and operable in accordance with any of the teachings of U.S. Pat. No. 4,805,823; U.S. Pat. No. 5,415,334; U.S. Pat. No. 5,465,895; U.S. Pat. No. 5,597,107; U.S. Pat. No. 5,632,432; U.S. Pat. No. 5,673,840; U.S. Pat. No. 5,704,534; U.S. Pat. No. 5,814,055; U.S. Pat. No. 6,964,363; U.S. Pat. No. 6,978,921; U.S. Pat. No. 6,988,649; U.S. Pat. No. 7,000,818; U.S. Pat. No. 7,111,769; U.S. Pat. No. 7,143,923; U.S. Pat. No. 7,303,108; U.S. Pat. No. 7,367,485; U.S. Pat. No. 7,380,695; U.S. Pat. No. 7,380,696; U.S. Pat. No. 7,404,508; U.S. Pat. No. 7,434,715; U.S. Pat. No. 7,721,930; and/or U.S. Pat. No. 7,455,208. Additional exemplary modifications that may be provided for instrument (10) will be described in greater detail below. Various suitable ways in which the below teachings may be incorporated into instrument (10) will be apparent to those of ordinary skill in the art. Similarly, various suitable ways in which the below teachings may be combined with various teachings of the patents cited herein will be apparent to those of ordinary skill in the art. It should also be understood that the below teachings are not limited to instrument (10) or devices taught in the patents cited herein. The below teachings may be readily applied to various other kinds of instruments, including instruments that would not be classified as surgical staplers. Various other suitable devices and settings in which the below teachings may be applied will be apparent to those of ordinary skill in the art in view of the teachings herein.

### II. Exemplary Interiorly Coated Surgical Staple

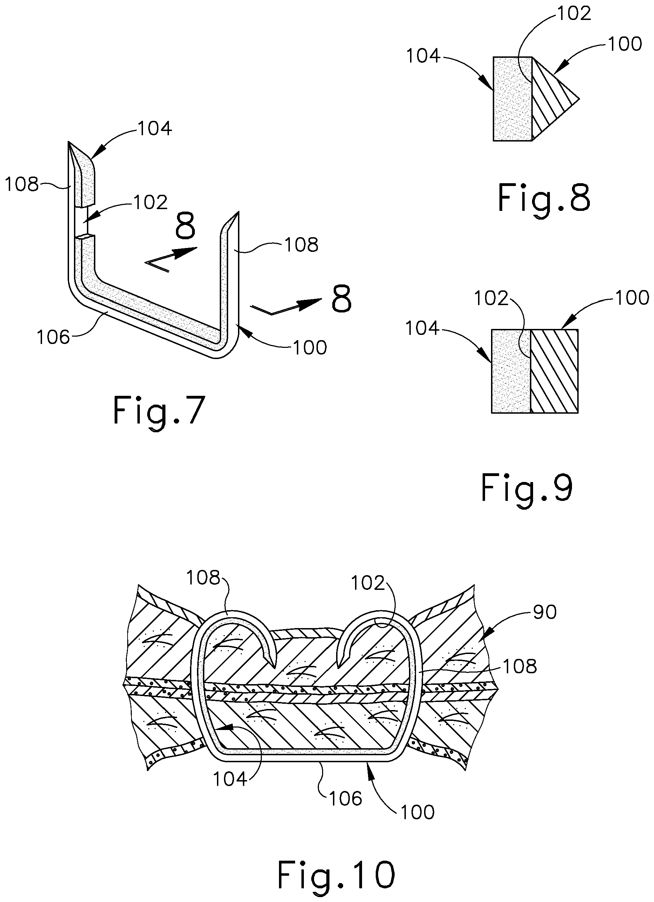

FIG. 7 shows an exemplary surgical staple (100) that is coated with localized adjunct coating (104) on a roughened interior surface (102). Surgical staple (100) may have a triangular wire form, as shown in FIG. 8; a rectangular wire form, as shown in FIG. 9; or any other wire form shape as will be understood by one of ordinary skill in the art in view of the teachings herein. Surgical staple (100) includes crown (106) having ends, from each of which a respective leg of pair of legs (108) extends substantially transversely and perpendicularly when staple (100) is in a first, pre-deployment form. Crown (106) and the pair of legs (108) comprise a material selected from at least one of iron, nickel titanium alloy, stainless steel, or titanium. Of course, any other suitable material or combination of materials may be used.

When surgical staple (100) is driven into tissue by a surgical instrument, such as by instrument (10) in the manner disclosed above, surgical staple (100) will compress, connect, and retain such tissue as shown in FIG. 10. In the second, post-deployment form shown in FIG. 10, each of legs (108) are bowed towards one another to curve around a top surface of an upper layer of tissue (90) and penetrate into the top surface of tissue (90) while crown (106) grasps a bottom surface of a lower layer of tissue (90), allowing crown (106) and legs (108) of staple (100) to compress, connect, and retain the layers of tissue (90) after staple (100) is deployed from instrument (10). Interior coating (104) of surgical staple (100) will contact the compressed tissue and release onto the tissue to assist with tissue repair, such as by acting as a hemostatic agent allowing for blood coagulation, which reduces the amount of bleeding at the surgical site.

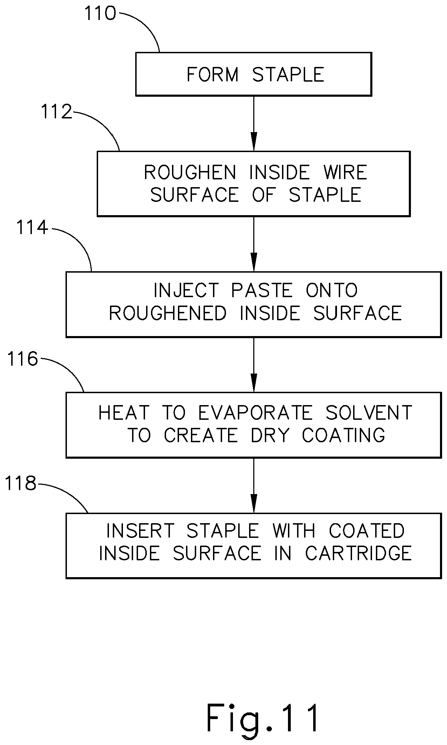

An exemplary method of forming the exemplary staple of FIG. 7 is shown in FIG. 11. The wire form of staple (100) is formed (110) according to processes known to those of ordinary skill in the art, such as those disclosed, by way of example only, by at least some of the teachings of U.S. Patent No. 6,638,297, entitled "Surgical Staple", issued October 28, 2003, the disclosure of which is incorporated by referenced herein. An inside or interior surface (102) of the wire surface of staple (100) is roughened (112) by a forming anvil, for example, which may act in a stamping process to roughen interior surface (102). As another merely illustrative example, an intermediate coating may be applied to interior surface (102) to provide roughening. For instance, such an intermediate coating may include grit. Suitable materials for such an intermediate coating will be apparent to those of ordinary skill in the art in view of the teachings herein. Other processes to roughen interior surface (102) may include alternative compression forming operations, spin forming operations, and/or grinding operations, for example, and other such metal forming processes and/or chemical roughening processes apparent to those of ordinary skill in the art in view of the teachings herein to roughen metal surfaces, all of which are within the scope of this disclosure.

A secondary molding process is used to inject (114) paste onto the roughened interior surface (102) of staple (100). The paste may comprise, for example, bovine collagen paste or another adjunct. For example, fibrin or thrombin may be used. The hemostatic abilities of such adjuncts may also contribute to the use of such adjuncts as adhesives and sealants. The agents may assist to coagulate blood at a surgical site which allows tissue surrounding such blood to stick together and may prevent leaks along the stapled tissue site, for example.

Such adjuncts or reagents may include but are not limited to medical fluid or paste components capable of being applied to interior surface (102) of staple (100) and then being heated to form a dry coating, as described below, such as thrombin, platelet poor plasma (PPP) platelet rich plasma (PRP), starch, chitosan, alginate, fibrin, polysaccharide, cellulose, collagen, bovine collagen, gelatin-resorcin-formalin adhesive, oxidized cellulose, mussel-based adhesive, poly (amino acid), agarose, amylose, hyaluronan, polyhydroxybutyrate (PHB), hyaluronic acid, poly(vinyl pyrrolidone) (PVP), poly(vinyl alcohol) (PVA), polylactide (PLA), polyglycolide (PGA), polycaprolactone (PCL), and their copolymers, VICRYL® (Ethicon, Inc., Somerville, N.J.), MONOCRYL material, PANACRYL (Ethicon, Inc., Somerville, N.J.), and/or any other material suitable to be mixed with biological material and introduced to a wound or defect site, including combinations of materials. Other suitable compounds, materials, substances, etc., that may be used in a medical fluid or paste will be apparent to those of ordinary skill in the art in view of the teachings herein.

In some versions, a medical fluid may be suspended in a biocompatible carrier. Suitable carriers may include, for example, a physiological buffer solution, a flowable gel solution, saline, and water. In the case of gel solutions, the tissue repair composition may be in a flowable gel form prior to delivery at the target site, or may form a gel and remain in place after delivery at the target site. Flowable gel solutions may comprise one or more gelling materials with or without added water, saline, or a physiological buffer solution. Suitable gelling materials include biological and synthetic materials. Exemplary gelling materials include the following: proteins such as collagen, collagen gel, elastin, thrombin, fibronectin, gelatin, fibrin, tropoelastin, polypeptides, laminin, proteoglycans, fibrin glue, fibrin clot, platelet rich plasma (PRP) clot, platelet poor plasma (PPP) clot, self-assembling peptide hydrogels, Matrigel or atelocollagen; polysaccharides such as pectin, cellulose, oxidized regenerated cellulose, chitin, chitosan, agarose, or hyaluronic acid; polynucleotides such as ribonucleic acids or deoxyribonucleic acids; other materials such as alginate, cross-linked alginate, poly(N-isopropylacrylamide), poly(oxyalkylene), copolymers of poly(ethylene oxide)-poly(propylene oxide), poly(vinyl alcohol), polyacrylate, or monostearoyl glycerol co-Succinate/polyethylene glycol (MGSA/PEG) copolymers; and combinations of any of the foregoing. In addition to providing a flowable carrier solution for tissue fragments, a gelling agent(s) may also act as an adhesive that anchors the tissue repair composition at the target site. In some versions, an additional adhesive anchoring agent may be included in the tissue repair composition or medical fluid. Also, one or more cross-linking agents may be used in conjunction with one or more gelling agents in order to cross-link the gelling agent.

Referring back to FIG. 11, staple (100), including the injected paste, is then heated (116) to evaporate solvent of the paste, resulting in dry coating (104) of adjunct that is localized on interior surface (102) of staple (100). Coated staple (100) is then inserted (118) into a cartridge, such as cartridge (37) described above, for use with an instrument capable of driving staple (100) into tissue (such as tissue (90) as shown in FIG. 6). While just one coating (104) is provided in this particular example, it should be understood that more than one coating (104) may be provided. For instance, interior surface (102) may receive several coatings (104) of the same material and/or different coatings of different materials (104). As one merely illustrative example, interior surface may receive a first coating (104) of a therapeutic agent or coagulant; then a second coating (104) that protects the first coating (104), with the second coating (104) being configured to disintegrate upon becoming wet, to thereby protect the first coating (104) until staple (100) is deployed in tissue. It should also be understood that layers of coatings (104) may provide bioreactive expandability or compliance, resulting in residual pressure applied (e.g., at a microscale) to adjacent tissue. Other suitable selections, properties, and combinations of coatings (104) will be apparent to those of ordinary skill in the art in view of the teachings herein.

In use, as the entire surface of staple (100) is not coated but rather coating (104) is localized and applied to interior surface (102) of staple (100), staple (100) will maintain a diameter such that staple (100) will fit into an existing staple pocket of cartridge (37), for example, which is also used for staples (47), as described above. Further, localized interior coating (104) of staple (100) may assist to reduce friction that may otherwise result via a fully coated staple, which may contribute to staple forming issues and an increase in force necessary to fire the staples through tissue. Additionally, less waste may occur with localized interior coating (104) disclosed herein. With a fully coated staple, contact between cartridge (37) and anvil (18) may cause coating on exterior surfaces of the staple to be prematurely removed and expelled onto instrument (10) rather than tissue (90), for example.

It should be understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures.

Versions of described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the invention as defined by the appended claims. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A medical fastener (100) having a first, pre-deployment shape for loading into a surgical instrument, and a second, post-deployment shape for connecting tissue together, the fastener comprising:
(a) a crown (106) including a pair of ends;
(b) a pair of legs (108), each leg (108) extending substantially transversely from a respective end of the crown (106) in the first, pre-deployment shape;
(c) a fastener interior surface (102) defined by interior surfaces of the crown (106) and the pair of legs (108);
(d) a fastener exterior surface defined by exterior surfaces of the crown (106) and the pair of legs (108); and **characterized in that**
(e) at least one coating (104) of a tissue repair composition is applied only to the interior surfaces of the crown (106) and the pair of legs (108), wherein the fastener exterior surface is uncoated.

2. The fastener (100) of claim 1, wherein the fastener (100) in the first, pre-deployment shape has a substantially rectangular cross-sectional shape or a substantially triangular cross-sectional shape.

3. The fastener (100) of claim 1, wherein the coating (104) comprises at least one of a hemostatic agent, a sealant, or an adhesive.

4. The fastener (100) of claim 1, wherein the coating (104) comprises fibrin or thrombin.

5. The fastener (100) of claim 1, wherein the coating (104) comprises a bovine collagen paste.

6. The fastener (100) of claim 1, wherein the interior surface (102) is roughened.

7. The fastener (100) of claim 1, wherein the crown (106) and the pair of legs (108) comprise a material selected from at least one of the following materials: iron, nickel titanium alloy, stainless steel, and titanium.

8. The fastener (100) of claim 1, wherein the coating (104) is a paste when applied to the fastener interior surface (102).

9. The fastener (100) of claim 1, wherein the coating (104) is a dry coating after application to the fastener interior surface (102).

10. The fastener (100) of claim 1, wherein the coating (104) is reactable with tissue when the medical fastener (100) is in the second, post-deployment shape for connecting tissue together.

11. A method of forming the fastener (100) of claim 1 by coating a pre-formed medical fastener having a first, pre-deployment shape for loading into a staple cartridge (37) of a surgical instrument (10), and a second, post-deployment shape for connecting tissue together, the method comprising the steps of:
(a) roughening an interior surface (102) of the fastener;
(b) injecting material onto the roughened interior surface (102);
(c) heating the injected material; and
(d) inserting the fastener (100) being a coated staple (100) into the staple cartridge (37).

12. The method of claim 11, wherein the step of heating the injected material comprises evaporating solvent of the material and creating a dry coating.

13. The method of claim 11, wherein the fastener (100) comprises a crown (106) and a pair of legs (108), each leg (108) substantially transversely extending from a respective end of the crown (106) in the first, pre-deployment shape.

14. The method of claim 11, wherein the step of injecting material onto the roughened interior surface (102) comprises injecting a hemostatic agent onto the roughened interior surface (102).

15. A surgical instrument (10) comprising a handle portion (20), a shaft (23) housing a firing bar (14), an end effector (12) comprising an anvil (18), a lower jaw (16), and a stapling and severing mechanism responsive to a longitudinal closing motion produced by the handle portion (20) and the shaft (23), the lower jaw (16) configured to receive a cartridge (37) when in an open position, the cartridge (37) comprising:
(a) a housing;
(b) a plurality of staples (47) disposed in the housing, each staple (47) having a first, pre-deployment shape for loading into a surgical instrument, and a second, post-deployment shape for connecting tissue together, each staple (47) being in accordance with the fastener (100) of claim 1;
wherein the stapling and severing mechanism is operable to drive the plurality of staples (47) toward the anvil (18) to form the second, post-deployment shape for each staple (47).

## Patentansprüche

1. Medizinisches Befestigungsmittel (100) mit einer ersten Gestalt vor der Freisetzung zum Laden in ein chirurgisches Instrument und einer zweiten Gestalt nach der Freisetzung zum Verbinden von Gewebe miteinander, wobei das Befestigungsmittel das Folgende umfasst:
(a) eine Krone (106) mit zwei Enden;
(b) zwei Schenkel (108), wobei sich jeder Schenkel (108) im Wesentlichen quer von einem jeweiligen Ende der Krone (106) in der ersten Gestalt vor der Freisetzung erstreckt;
(c) eine Befestigungsmittelinnenfläche (102), die von Innenflächen der Krone (106) und der zwei Schenkel (108) definiert wird;
(d) eine Befestigungsmittelaußenfläche, die von Außenflächen der Krone (106) und der zwei Schenkel (108) definiert wird, und **dadurch gekennzeichnet, dass**
(e) zumindest eine Beschichtung (104) einer Gewebereparaturzusammensetzung nur auf den Innenflächen der Krone (106) und der zwei Schenkel (108) aufgebracht ist, wobei die Befestigungsmittelaußenfläche unbeschichtet ist.

2. Befestigungsmittel (100) nach Anspruch 1, wobei das Befestigungsmittel (100) in der ersten Gestalt vor der Freisetzung eine im Wesentlichen rechteckige Querschnittsgestalt oder eine im Wesentlichen dreieckige Querschnittsgestalt aufweist.

3. Befestigungsmittel (100) nach Anspruch 1, wobei die Beschichtung (104) zumindest eines von einem hämostatischen Mittel, einem Dichtungsmittel oder einem Klebstoff umfasst.

4. Befestigungsmittel (100) nach Anspruch 1, wobei die Beschichtung (104) Fibrin oder Thrombin umfasst.

5. Befestigungsmittel (100) nach Anspruch 1, wobei die Beschichtung (104) eine Rinderkollagenpaste umfasst.

6. Befestigungsmittel (100) nach Anspruch 1, wobei die Innenfläche (102) aufgeraut ist.

7. Befestigungsmittel (100) nach Anspruch 1, wobei die Krone (106) und die zwei Schenkel (108) ein Material umfassen, das unter zumindest einem der folgenden Materialien ausgewählt ist: Eisen, Nickel-Titan-Legierung, Edelstahl und Titan.

8. Befestigungsmittel (100) nach Anspruch 1, wobei die Beschichtung (104) eine Paste ist, wenn sie auf die Befestigungsmittelinnenfläche (102) aufgebracht wird.

9. Befestigungsmittel (100) nach Anspruch 1, wobei die Beschichtung (104) eine trockene Beschichtung ist, nachdem sie auf die Befestigungsmittelinnenfläche (102) aufgebracht wurde.

10. Befestigungsmittel (100) nach Anspruch 1, wobei die Beschichtung (104) mit Gewebe reagieren kann, wenn sich das medizinische Befestigungsmittel (100) in der zweiten Gestalt nach der Freisetzung zum Verbinden von Gewebe miteinander befindet.

11. Verfahren zur Bildung der Befestigungsmittel (100) nach Anspruch 1, durch Beschichtung eines vorgeformten medizinischen Befestigungsmittels mit einer ersten Gestalt vor der Freisetzung zum Laden in ein Klammermagazin (37) eines chirurgischen Instruments (10), und einer zweiten Gestalt nach der Freisetzung zum Verbinden von Gewebe miteinander, wobei das Verfahren die folgenden Schritte umfasst:
(a) Aufrauen einer Innenfläche (102) des Befestigungsmittels;
(b) Einspritzen von Material auf die aufgeraute Innenfläche (102);
(c) Erhitzen des eingespritzten Materials; und
(d) Einsetzen des Befestigungsmittels (100), das eine beschichtete Klammer (100) ist, in das Klammermagazin (37).

12. Verfahren nach Anspruch 11, wobei der Schritt des Erhitzens des eingespritzten Materials das Abdampfen von Lösungsmittel aus dem Material und das Erzeugen einer trockenen Beschichtung umfasst.

13. Verfahren nach Anspruch 11, wobei das Befestigungsmittel (100) eine Krone (106) und zwei Schenkel (108) umfasst, wobei sich jeder Schenkel (108) im Wesentlichen quer von einem jeweiligen Ende der Krone (106) in der ersten Gestalt vor der Freisetzung erstreckt.

14. Verfahren nach Anspruch 11, wobei der Schritt des Einspritzens von Material auf die aufgeraute Innenfläche (102) das Einspritzen von hämostatischem Mittel auf die aufgeraute Innenfläche (102) umfasst.

15. Chirurgisches Instrument (10), umfassend einen Griffabschnitt (20), einen Schaft (23), in dem ein Abfeuerstab (14) untergebracht ist, einen Endeffektor (12), der einen Amboss (18) umfasst, eine untere Backe (16) und einen Klammer- und Durchtrennmechanismus, der auf eine längs gerichtete Schließbewegung anspricht, die vom Griffabschnitt (20) und dem Schaft (23) erzeugt wurde, wobei die untere Backe (16) dazu ausgelegt ist, in einer offenen Position ein Magazin (37) aufzunehmen, wobei das Magazin (37) das Folgende umfasst:
(a) ein Gehäuse;
(b) eine Mehrzahl von Klammern (47), die im Gehäuse angeordnet sind, wobei jede Klammer (47) eine erste Gestalt vor der Freisetzung zum Laden in ein chirurgisches Instrument und eine zweite Gestalt nach der Freisetzung zum Verbinden von Gewebe miteinander aufweist, wobei jede Klammer (47) dem Befestigungsmittel (100) nach Anspruch 1 entspricht;
wobei der Klammer- und Durchtrennmechanismus dazu betätigbar ist, die Mehrzahl von Klammern (47) zum Amboss (18) zu treiben, um die zweite Gestalt nach der Freisetzung für jede Klammer (47) zu bilden.

## Revendications

1. Attache médicale (100) possédant une première forme de prédéploiement pour un chargement dans un instrument chirurgical, et une seconde forme de postdéploiement pour relier des tissus ensemble, l'attache comprenant :
(a) une couronne (106) comprenant une paire d'extrémités ;
(b) une paire de pattes (108), chaque patte (108) s'étendant sensiblement transversalement depuis une extrémité respective de la couronne (106) dans la première forme de prédéploiement ;
(c) une surface intérieure (102) d'attache définie par des surfaces intérieures de la couronne (106) et des deux pattes (108) ;
(d) une surface extérieure d'attache définie par des surfaces extérieures de la couronne (106) et des deux pattes (108) ; et **caractérisée en ce que**
(e) au moins un revêtement (104) d'une composition de réparation tissulaire est appliqué uniquement sur les surfaces intérieures de la couronne (106) et des deux pattes (108), la surface extérieure d'attache n'étant pas revêtue.

2. Attache (100) selon la revendication 1, l'attache (100) dans la première forme de prédéploiement ayant une forme de coupe transversale sensiblement rectangulaire ou une forme de coupe transversale sensiblement triangulaire.

3. Attache (100) selon la revendication 1, dans laquelle le revêtement (104) comprend au moins un parmi un agent hémostatique, un agent d'étanchéité ou un adhésif.

4. Attache (100) selon la revendication 1, dans laquelle le revêtement (104) comprend de la fibrine ou de la thrombine.

5. Attache (100) selon la revendication 1, dans laquelle le revêtement (104) comprend une pâte à base de collagène bovin.

6. Attache (100) selon la revendication 1, dans laquelle la surface intérieure (102) est rugosifiée.

7. Attache (100) selon la revendication 1, dans laquelle la couronne (106) et les deux pattes (108) comprennent un matériau sélectionné parmi au moins un des matériaux suivants : le fer, l'alliage de nickel et de titane, l'acier inoxydable, et le titane.

8. Attache (100) selon la revendication 1, dans laquelle le revêtement (104) est une pâte lorsqu'il est appliqué sur la surface intérieure (102) d'attache.

9. Attache (100) selon la revendication 1, dans laquelle le revêtement (104) est un revêtement sec après application sur la surface intérieure (102) d'attache.

10. Attache (100) selon la revendication 1, dans laquelle le revêtement (104) peut réagir avec le tissu lorsque l'attache médicale (100) est dans la seconde forme de postdéploiement pour relier des tissus ensemble.

11. Procédé de formation de l'attache (100) selon la revendication 1 par le revêtement d'une attache médicale préformée possédant une première forme de prédéploiement pour un chargement dans une cartouche (37) d'agrafes d'un instrument chirurgical (10), et une seconde forme de postdéploiement pour relier des tissus ensemble, le procédé comportant les étapes consistant à :
(a) rugosifier une surface intérieure (102) de l'attache ;
(b) injecter un matériau sur la surface intérieure rugosifiée (102) ;
(c) chauffer le matériau injecté ; et
(d) introduire l'attache (100) qui est une agrafe revêtue (100) dans la cartouche (37) d'agrafes.

12. Procédé selon la revendication 11, dans lequel l'étape consistant à chauffer le matériau injecté consiste à évaporer un solvant du matériau et à créer un revêtement sec.

13. Procédé selon la revendication 11, dans lequel l'attache (100) comprend une couronne (106) et une paire de pattes (108), chaque patte (108) s'étendant sensiblement transversalement depuis une extrémité respective de la couronne (106) dans la première forme de prédéploiement.

14. Procédé selon la revendication 11, dans lequel l'étape consistant à injecter un matériau sur la surface intérieure rugosifiée (102) consiste à injecter un agent hémostatique sur la surface intérieure rugosifiée (102).

15. Instrument chirurgical (10) comprenant une partie poignée (20), un arbre (23) renfermant une barre de déclenchement (14), un effecteur terminal (12) comprenant une enclume (18), une mâchoire inférieure (16), et un mécanisme d'agrafage et de détachement sensible à un mouvement longitudinal de fermeture produit par la partie poignée (20) et l'arbre (23), la mâchoire inférieure (16) étant conçue pour recevoir une cartouche (37) en position ouverte, la cartouche (37) comprenant :
(a) un logement ;
(b) une pluralité d'agrafes (47) disposées dans le logement, chaque agrafe (47) possédant une première forme de prédéploiement pour un chargement dans un instrument chirurgical, et une seconde forme de postdéploiement pour relier des tissus ensemble, chaque agrafe (47) étant conforme à l'attache (100) selon revendication 1 ;
le mécanisme d'agrafage et de détachement pouvant fonctionner pour amener la pluralité d'agrafes (47) vers l'enclume (18) à former la seconde forme postdéploiement pour chaque agrafe (47).
